# EUROPÄISCHE PATENTSCHRIFT

(11) **EP 1 856 502 B1**
(45) Veröffentlichungstag und Bekanntmachung des Hinweises auf die Patenterteilung: **03.06.2015**
(21) Anmeldenummer: 06723126.6
(22) Anmeldetag: 27.02.2006
(51) Int. Cl.: G01N 1/22

(54) **VORRICHTUNG ZUM MESSEN DES GASGEHALTES IN EINER METALLSCHMELZE**
DEVICE FOR MEASURING THE GAS CONTENT IN A METAL MELT
DISPOSITIF DE MESURE DE LA TENEUR EN GAZ DANS UN BAIN DE METAL EN FUSION

(30) Priorität: 09.03.2005 DE 102005011181
(43) Veröffentlichungstag der Anmeldung: 21.11.2007
(73) Patentinhaber: Heraeus Electro-Nite International N.V., 3530 Houthalen (BE)
(72) Erfinder: GERITS, Erik, B-3600 Genk (BE); PLESSERS, Jacques, B-3530 Houthalen-Helchteren (BE); SWENNEN, Jos, B-3670 Meeuwen-Gruitrode (BE)
(74) Vertreter: Kühn, Hans-Christian
(86) Internationale Anmeldenummer: PCT/EP2006/001765
(87) Internationale Veröffentlichungsnummer: WO 2006/094668

(56) Entgegenhaltungen:
- EP-A- 0 295 798
- EP-A- 0 307 430
- DE-A1- 2 423 783
- US-B1- 6 216 526

## Beschreibung

Die Erfindung betrifft eine Vorrichtung zum Messen des Gasgehaltes in einer Metallschmelze, mit einem einen Gassammelkörper aufweisenden Eintauchende, einer an dem Eintauchende mündenden Gaszuleitung und einer Gasableitung für die den Gassammelkörper durchdringenden Gase sowie ihre Verwendung.

Derartige Vorrichtungen sind aus EP 307 430 B1 bekannt. Die hier beschriebenen Vorrichtungen sind geeignet zur Messung des Gasgehaltes, insbesondere von Wasserstoff, beispielsweise in einer Stahlschmelze. Dabei ist der Gassammelkörper aus porösem Stein hergestellt. Bei verschiedenen Schmelzen kann es vorkommen, dass die Messung behindert wird, dadurch, dass die Öffnungen des Körpers verstopft werden oder die Oberfläche des Körpers keinen ausreichenden Kontakt mit der Metallschmelze erhält.

Eine ähnliche Vorrichtung ist auch US 6,216,526 B1 bekannt. Diese Vorrichtung weist ein Quarzglasrohr auf, in dem die Schmelze gesammelt wird. Die Metallschmelze kann dann durch einen für sie durchlässigen Stopfen in das Innere der Eintauchsonde eindringen. Diese Stopfen aus Aluminiumoxid soll Verunreinigungen in der Metallschmelze zurückhalten.

Aus DE 38 74 423 T2 (EP 295 798 B1) ist eine Sonde zur Bestimmung der Konzentration eines Gases in geschmolzenem Metall bekannt, die einen Gassammelkörper umfasst, wobei der Gassammelkörper eine Gaszuleitung und eine Gasableitung für die den Gassammelkörper durchdringenden Gase aufweist. Der Gassammelkörper besteht aus Aluminiumoxid. Aus DT 24 23 783 A1 ist ein Eintauchsensor zur Messung von Sauerstoff in Metallschmelzen bekannt mit einem Festelektrolyten mit Spinellstruktur.

Der Erfindung liegt die Aufgabe zugrunde, die vorhandenen Vorrichtungen zu verbessern und insbesondere eine Blockierung des Gassammelkörpers zu vermeiden.

Die Aufgabe wird erfindungsgemäß durch die Merkmale des unabhängigen Anspruchs gelöst. Vorteilhafte Ausgestaltungen sind in den Unteransprüchen angegeben.

Dadurch, dass der Gassammelkörper solche Materialien enthält, die in Kontakt mit der Metallschmelze keine flüssigen Reaktionsprodukte bilden, kann die Oberfläche des Gassammelkörpers nicht mit Fremdmaterialien belegt werden, so dass der Kontakt der Metallschmelze mit dem Gassammelkörper gewährleistet ist und dadurch ein Gasaustausch ermöglicht wird. Insbesondere ist es vorteilhaft, dass der Anteil der Materialien, die in Kontakt mit der Metallschmelze keine flüssigen Reaktionsprodukte bilden, mindestens 80 Gew.-%, vorzugsweise mindestens 90 Gew.-%, bezogen auf den Gassammelkörper, beträgt. Zweckmäßig ist es außerdem, dass der Gassammelkörper ein poröser Körper ist, wobei die Porosität vorzugsweise etwa 50 % beträgt.

Vorteilhafterweise enthält der Gassammelkörper Magnesiumoxid und/oder Aluminiumoxid und/oder Chromoxid. Insbesondere kann der Gassammelkörper ein Material der Formel AB₂O₄ enthalten, wobei A ein Metall vorzugsweise der Gruppe Mg, Fe, Mn und B ein weiteres Metall vorzugsweise der Gruppe Al, Cr, Fe, V ist. Das Material der Formel AB₂O₄ weist Spinehstruktur auf. Dieses wird aus Material des Gassammelkörpers erst beim Eintauchen oder während des Aufenthalts des Gassammelkörpers in Eisen- oder Stahlschmelze gebildet Zur Bildung der Struktur AB₂O₄ können vorzugsweise die oben genannten Metalle verwendet werden.

Erfindungsgemäß ist es, dass die keine flüssigen Reaktionsprodukte bildenden Materialien mindestens einen Teil der Oberfläche des Gassammelkörpers als Oberflächenschicht bilden, nämlich mindestens einen Teil der Oberfläche des Gassammelkörpers, der zur Berührung mit der Metallschmelze vorgesehen ist. Die Oberflächenschicht weist eine Dicke von etwa 0,3 bis 5 mm auf.

Die Vorrichtung kann erfindungsgemäß verwendet werden in Stahlschmelzen mit einem Sauerstoffgehalt von mindestens 100 ppm und/oder einem Gehalt an Schwefel und/oder Magnesium und/oder Silizium von mindestens 0,1 Gew.-%. Die Vorrichtung kann erfindungsgemäß auch verwendet werden zur Messung von Wasserstoff, Stickstoff, Kohlenmonoxid und/oder Kohlendioxid in Stahlschmelzen.

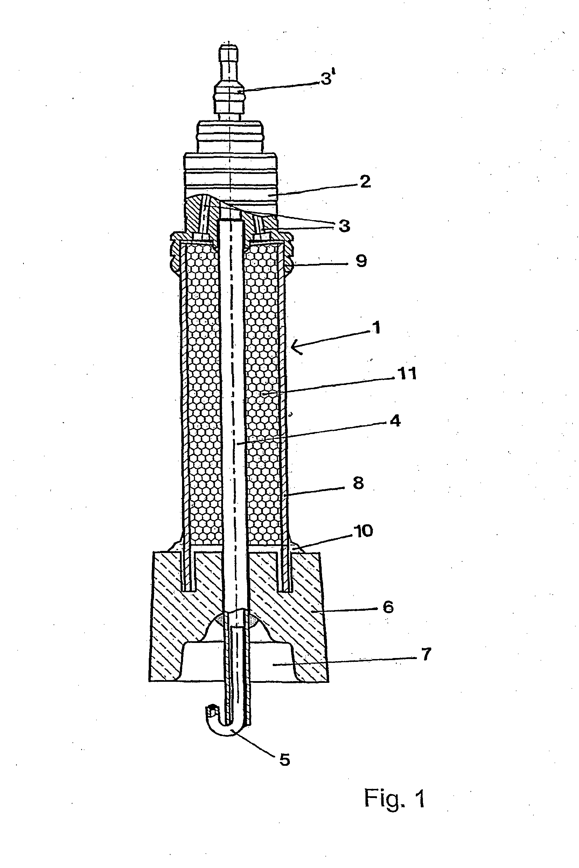

Nachfolgend wird ein Ausführungsbeispiel der Erfindung anhand einer Zeichnung erläutert. In der Zeichnung zeigt
Figur 1 das Eintauchende einer erfindungsgemäßen Vorrichtung, im Schnitt

Der prinzipielle Aufbau einer Vorrichtung zum Messen des Gasgehaltes in Metallschmelzen ist beispielsweise aus EP 307 430 B1 bekannt. Figur 1 der EP 307 430 B1 zeigt in Verbindung mit der Beschreibung den Messaufbau einschließlich des Eintauchendes mit einem Gassammelkörper. Auf diesen prinzipiellen Aufbau kann auch die vorliegende Erfindung zurückgreifen. Der Messvorgang ist ebenfalls beispielsweise in EP 307 430 B1 beschrieben.

Das Eintauchende 1 wird an einem Befestigungsstutzen 2 mit einem Trägerrohr verbunden, mit dessen Hilfe der Tauchvorgang erfolgt. Der Befestigungsstutzen enthält mehrere Gasanschlüsse 3; 3', wobei der zentrisch angeordnete Gasanschlussstutzen 3' durch eine Gaszuleitung 4 Trägergas in die Metallschmelze einleitet. Die Gaszuleitung 4 besteht im wesentlichen aus einem Quarzrohr, welches an seinem Eintauchende in ein weiteres, gebogenes Quarzrohr 5 mündet, dessen Mündung in Richtung des Gassammelkörpers 6 gerichtet ist. Der Gassammelkörper 6 weist eine glockenförmige Vertiefung 7 um die Gaszuleitung 4 herum auf. Die Mündung des Quarzrohres 5 ist zu der Vertiefung 7 hin gerichtet, so dass der Gasstrom aus der Zuleitung 4 zu der Vertiefung 7 hin erfolgt. Dabei wird Gas aus der Metallschmelze aufgenommen und mit dem Trägergas durch den Körper des Eintauchendes 1 hindurch zu den Gasanschlüssen 3 geführt. Von dort erfolgt eine Weiterleitung zu den entsprechenden Messeinrichtungen.

Zwischen dem Befestigungsstutzen 2 und dem Gassammelkörper 6 weist das Eintauchende 1 ein Quarzrohr 8 auf, welches am Befestigungsstutzen 2 und am Gassammelkörper 6 mittels Klebstoff 9 oder Zement 10 befestigt ist. Das Quarzrohr 8 ist mit Aluminiumoxid 11 gefüllt, welches zum einen die Gaszuleitung 4 fixiert und zum anderen die Weiterleitung des Trägergases mit dem zu messenden Gas ermöglicht.

Der Gassammelkörper 6 weist eine Porosität von etwa 50 % auf, so dass das Gas aus der Stahlschmelze aufgenommen werden kann. Der mittlere Porendurchmesser beträgt etwa 40 µm. Die Zusammensetzung des Gassammelkörpers gewährleistet, dass die Poren nicht irgendwie verstopft oder verdeckt werden, sondern offen bleiben, so dass das Gas ungehindert eindringen kann.

In einem Beispiel wird der Gassammelkörper 6 geformt aus gepresstem und gesintertem Al₂O₃ -Korund, danach wird er getränkt und imprägniert in einer MgO-Suspension auf Wasserbasis, wobei eine Oberflächenschicht entsteht. Der Gassammelkörper 6 wird anschließend getrocknet. Die Dicke der Oberflächenschicht beträgt je nach Behandlung etwa 0,3 bis 5 mm, vorzugsweise 1 bis 3 mm. Die Schicht ist in der Zeichnung nicht dargestellt.

## Patentansprüche

1. Vorrichtung zum Messen des Gasgehaltes in einer Metallschmelze, mit einem einen Gassammelkörper aufweisenden Eintauchende (1), einer an dem Eintauchende mündenden Gaszuleitung (4) und einer Gasableitung (3) für die den Gassammelkörper durchdringenden Gase, wobei der Gassammelkörper solche Materialien enthält, die in Kontakt mit der Metallschmelze keine flüssigen Reaktionsprodukte binden, **dadurch gekennzeichnet, dass** die keine flüssigen Reaktionsprodukte bildenden Materialien mindestens einen Teil der Oberfläche des Gassammelkörpers als Oberflächenschicht bilden, der zur Berührung mit der Metallschmelze vorgesehen ist und dass die Oberflächenschicht eine Dicke von etwa 0,3 bis 5 mm aufweist und dass der Gassammelkörper ein Material enthält, welches sich beim Tauchen der Vorrichtung in Eisen- oder Stahlschmelze in AB₂O₄ umwandelt, wobei A ein Metall der Gruppe Mg, Fe, Mn und B ein weiteres Metall der Gruppe Al, Cr, Fe, V ist.

2. Vorrichtung nach Anspruch 1, **dadurch gekennzeichnet, dass** der Anteil der Materialien, die in Kontakt mit der Metallschmelze keine flüssigen Reaktionsprodukte bilden, mindestens 80 Gew.-%, vorzugsweise mindestens 90 Gew.-%, bezogen auf den Gassammelkörper, beträgt.

3. Vorrichtung nach Anspruch 1 oder 2, **dadurch gekennzeichnet, dass** der Gassammelkörper ein poröser Körper ist.

4. Vorrichtung nach einem der Ansprüche 1 bis 3, **dadurch gekennzeichnet, dass** der Gassammelkörper Magnesiumoxid und/oder Aluminiumoxid und/oder Chromoxid enthält.

5. Verwendung einer Vorrichtung nach einem der Ansprüche 1 bis 4 zur Messung in Stahlschmelzen mit einem Sauerstoffgehalt von mindestens 100 ppm und/oder einem Gehalt an Schwefel und/oder Magnesium und/oder Silizium von mindestens 0,1 Gew.-%.

6. Verwendung einer Vorrichtung nach einem der Ansprüche 1 bis 4 zur Messung von Wasserstoff, Stickstoff, Kohlenmonoxid und/oder Kohlendioxid in Stahlschmelzen.

## Claims

1. Device for measuring the gas content in a molten metal, with an immersion end (1) comprising a gas collection body, a gas feed line (4) merging at the immersion end, and a gas discharge line (3) for the gases passing through the gas collection body, whereby the gas collection body contains materials which do not form liquid reaction products when in contact with the molten metal, **characterised in that** the materials that form no liquid reaction products form at least a part of the surface of the gas collection body in the form of a surface layer that is provided for contacting the molten metal, and **in that** the surface layer has a thickness of approximately 0.3 to 5 mm, and **in that** the gas collection body contains a material which converts into AB₂O₄ when the device is immersed in molten iron or molten steel, whereby A is a metal of the group Mg, Fe, Mn, and B is a further metal of the group Al, Cr, Fe, V.

2. Device according to claim 1, **characterised in that** the fraction of the materials that do not form liquid reaction products when they contact the molten metal is at least 80 % by weight, preferably at least 90 % by weight, with respect to the gas collection body.

3. Device according to claim 1 or 2, **characterised in that** the gas collection body is a porous body.

4. Device according to any one of the claims 1 to 3, **characterised in that** the gas collection body contains magnesium oxide and/or aluminium oxide and/or chromium oxide.

5. Use of a device according to any one of the claims 1 to 4 for measurements in molten steel with an oxygen content of at least 100 ppm and/or a content of sulfur and/or magnesium and/or silicon of at least 0.1 % by weight.

6. Use of a device according to any one of the claims 1 to 4 for hydrogen, nitrogen, carbon monoxide and/or carbon dioxide measurements in molten steel.

## Revendications

1. Dispositif de mesure de la teneur en gaz dans une fonte métallique, comprenant une extrémité d'immersion (1) présentant un corps de collecte de gaz, une arrivée de gaz (4) débouchant sur l'extrémité d'immersion et une évacuation de gaz (3) pour les gaz traversant le corps de collecte de gaz, sachant que le corps de collecte de gaz contient des matériaux ne formant pas de produits de réaction liquides en contact avec la fonte métallique, **caractérisé en ce que** les matériaux ne formant pas de produits de réaction liquides forment au moins une partie de la surface du corps de collecte de gaz en tant que couche superficielle, qui est prévue pour le contact avec la fonte métallique, et que la couche superficielle présente une épaisseur d'environ 0,3 à 5 mm et que le corps de collecte de gaz contient un matériau qui se transforme en AB₂O₄ lorsque le dispositif est plongé dans une fonte de fer ou d'acier, sachant qu'A est un métal du groupe Mg, Fe, Mn et B un métal supplémentaire du groupe Al, Cr, Fe, V.

2. Dispositif selon la revendication 1, **caractérisé en ce que** la teneur des matériaux ne formant pas de produits de réaction liquides en contact avec la fonte métallique, est d'au moins 80 % en poids, de préférence d'au moins 90 % en poids, rapporté au corps de collecte de gaz.

3. Dispositif selon la revendication 1 ou 2, **caractérisé en ce que** le corps de collecte de gaz est un corps poreux.

4. Dispositif selon l'une des revendications 1 à 3, **caractérisé en ce que** le corps de collecte de gaz contient de l'oxyde de magnésium et/ou de l'oxyde d'aluminium et/ou de l'oxyde de chrome.

5. Emploi d'un dispositif selon l'une des revendications 1 à 4 pour des mesures dans des fontes d'acier ayant une teneur en oxygène d'au moins 100 ppm et/ou une teneur en soufre et/ou en magnésium et/ou en silicium d'au moins 0,1 % en poids.

6. Emploi d'un dispositif selon l'une des revendications 1 à 4 pour des mesures d'hydrogène, d'azote, de monoxyde de carbone et/ou de dioxyde de carbone dans des fontes d'acier.
